# EUROPEAN PATENT APPLICATION

(11) **EP 1 147 764 A2**
(43) Date of publication of application: **24.10.2001**
(21) Application number: 01109611.2
(22) Date of filing: 18.04.2001
(51) Int. Cl.: A61K 7/48, C07D 311/30

(54) **Cosmetic composition**

(30) Priority: 19.04.2000 JP 2000117519
(71) Applicant: NOF CORPORATION, Tokyo (JP)
(72) Inventor: Ishida, Misaki, Amagasaki-shi, Hyogo (JP); Sato, Saori, Minamiashigara-shi, Kanagawa (JP); Hashizume, Ron, Abiko-shi, Chiba (JP); Hayashi, Shinji, Amagasaki-shi, Hyogo (JP)
(74) Representative: Koepe, Gerd L., Dipl.-Chem.

(57) **Abstract**

A cosmetic composition comprises 0.00005 to 10wt% of polymethoxyflavone having at least four methoxy groups. Cosmetic compositions in the form of lotions, milky lotions, oil-in-water creams, water-in-oil creams are preferable. Thus, the present invention provides cosmetic compositions that are excellent in the whitening effect, allow the skin to retain moisture for a long time, vitalize the skin, suppress wrinkles, and have excellent storage stability.

## Description

### BACKGROUND OF THE INVENTION

### 1. Field of the invention

The present invention relates to cosmetic compositions. More specifically, the present invention relates to cosmetic compositions that are excellent in whitening effects, effectively prevent "liver spots", "freckles" etc. that are caused by sunburn, allow the skin to retain moisture for a long time, vitalize the skin, suppress wrinkles, and are excellent in safety and storage stability.

### 2. Description of the prior art

When the skin is irradiated with ultraviolet rays (hereinafter referred to as UV), melanocytes in the skin are activated, so that synthesis of melanin is promoted by the enzyme tyrosinase as well as TRP1 and TRP2. Deposition of the produced melanin in the skin results in "liver spots" or "freckles", and therefore various cosmetic compositions, especially whitening cosmetics are used to prevent such melanin production. Moreover, the UV are found to promote oxidation of sebum cutaneum, cell membranes or the like and cause various skin disorders. In recent years, in particular, an increase in the amount of UV due to damage of the ozone layer is noted as a problem, and it is desired to prevent oxidation in the skin more effectively.

Ascorbic acid phosphate ester salts, hydroquinon derivatives, placental extracts, kojic acids, ellagic acid or the like are known as components exhibiting a whitening effect, and cosmetic compositions comprising these components are commonly used. Among these, at present, the components that are most commonly used are placental extracts, usually bovine placental extracts. However, in recent years, bovine spongiform encephalopathy has become worldwide problems. In Japan, use of bovine placental extracts is banned since December 2000. Therefore, a novel and effective whitening component is in demand.

On the other hand, in recent years, it has been reported that polyphenol or the like contained in plants has a high whitening effect, and cosmetic compositions employing it have been proposed. Furthermore, for example, Japanese Laid-Open Patent Publication (Tokkai) No. 6-16531 reports that flavanones have a whitening effect, and Japanese Laid-Open Patent Publication (Tokkai) No. 10-101543 reports that hydroxyflavones have a whitening effect. However, not only are the effects of these whitening components not sufficient yet, but also these components have poor storage stability, so that when they become finished products, various problems may arise over time. Furthermore, although these whitening components have a whitening effect, so-called anti skin-aging effects such as vitalizing the skin and preventing wrinkles cannot sufficiently be provided at present.

Therefore, there is a great demand for cosmetic compositions having an excellent whitening effect, providing a skin aging prevention effect, high safety in use, and excellent storage stability.

### SUMMARY OF THE INVENTION

Therefore, it is an object of the present invention to provide a cosmetic composition having a sufficient excellent whitening effect, a so-called anti skin-aging effect such as vitalizing the skin and preventing wrinkles, high safety in use, and excellent storage stability. This object can be achieved by blending a specific amount of a specific polymethoxyflavone compound.

A cosmetic composition of the present invention includes 0.00005 to 10 percent by weight (hereinafter referred to as wt%) of polymethoxyflavone represented by formula (I): 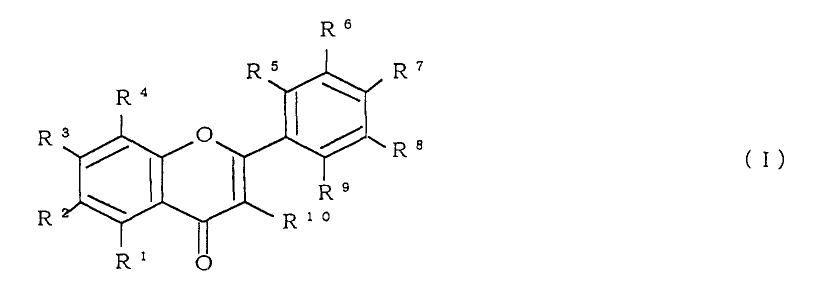 wherein each of R¹, R², R³, R⁴, R⁵, R⁶, R⁷, R⁸, R⁹, and R¹⁰ is selected from the group consisting of hydrogen atom, hydroxyl group, alkoxy group having 1 to 20 carbon atoms, alkyl group having 1 to 20 carbon atoms, alkenyl group having 2 to 20 carbon atoms, hydroxyalkyl group having 1 to 20 carbon atoms or a sugar residue, and at least four of R¹, R², R³, R⁴, R⁵, R⁶, R⁷, R⁸, R⁹, and R¹⁰ are methoxy groups.

In one preferred embodiment of the present invention, the polymethoxyflavone comprises at least one compound selected from the group consisting of compounds represented by formulae (II) to (V):

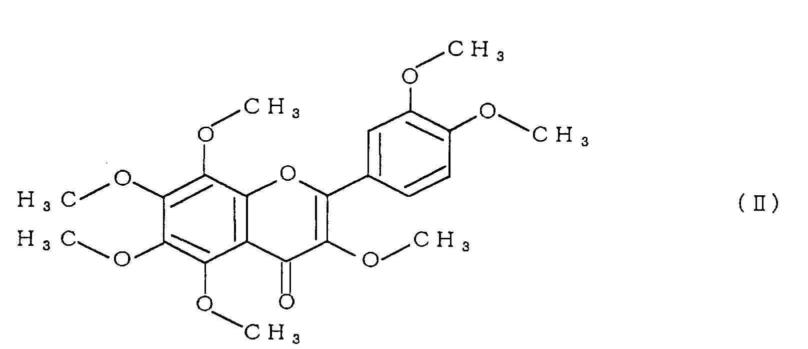

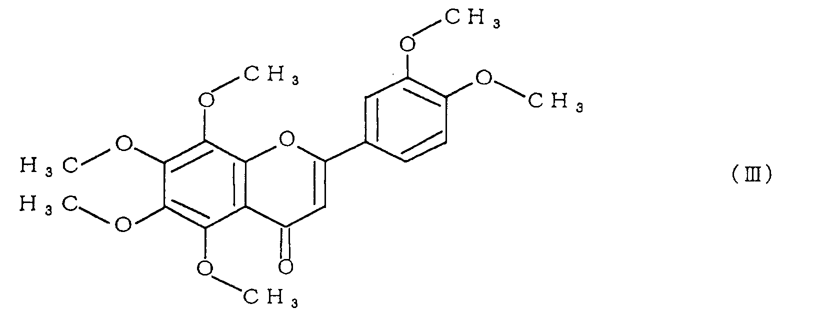

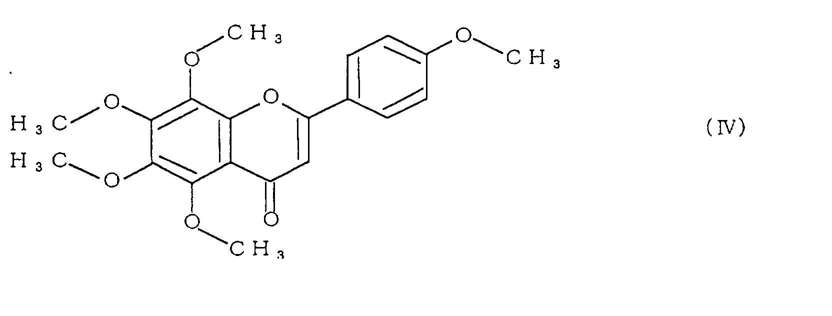

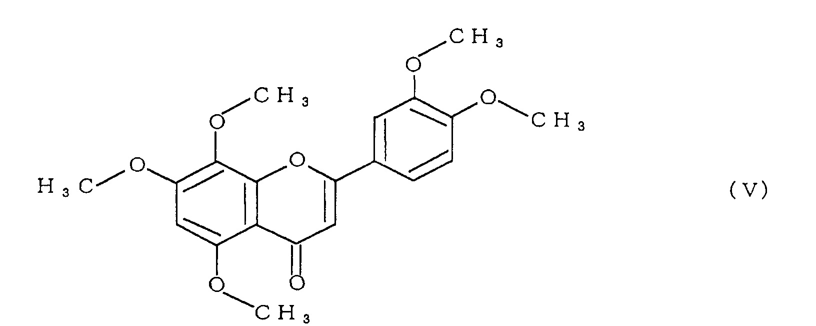

In one preferred embodiment of the present invention, the polymethoxyflavone comprises at least one compound selected from the group consisting of 5,6,7,8,3',4'-hexamethoxyflavone and 5,6,7,8,4'-pentamethoxyflavone.

According to another aspect of the present invention, a method for isolating and purifying polymethoxyflavone includes the steps of: subjecting peel of a plant of the Genus Citrus of the Family Rutaceae to extraction with at least one solvent selected from the group consisting of methanol, ethanol, propanol, butanol, ethyl acetate, acetone, propylene glycol, and 1, 3-butylene glycol to obtain an extract (S1); dissolving the extract (S1) in ethyl acetate, adding water thereto, stirring, separating into layers, removing a water layer, distilling off the ethyl acetate to obtain a dry solid product (S2); and dissolving the dry solid product (S2) in a solvent, and subjecting it to liquid column chromatography.

According to another aspect of the present invention, a method for isolating and purifying polymethoxyflavone comprising the steps of: subjecting peel of a plant of the Genus Citrus of the Family Rutaceae to extraction with at least one solvent selected from the group consisting of methanol, ethanol, propanol, butanol, ethyl acetate, acetone, propylene glycol, and 1, 3-butylene glycol to obtain an extract (S1); dissolving the extract (S1) in hexane and/or chloroform, removing a precipitate, distilling hexane and/or chloroform to obtain a dry solid product (S3); and dissolving the dry solid product (S3) in a solvent, and subjecting it to liquid column chromatography.

In one preferred embodiment of the present invention, the liquid column chromatography uses silica gel and/or alumina as a filler, and a mixed solution of hexane/ethanol in a volume proportion of 70/30 to 97/3 as an eluent.

Thus, the cosmetic compositions of the present invention has an excellent whitening effect and has excellent storage stability.

These and other advantages of the present invention will become apparent to those skilled in the art upon reading and understanding the following detailed description.

### BRIEF DESCRIPTION OF THE DRAWINGS

Fig. 1 is a diagram showing the ¹H-NMR of the compound represented by formula (III).
Fig. 2 is a diagram showing the ¹³C-NMR of the compound represented by formula (III).
Fig. 3 is a diagram showing the mass spectrum of the compound represented by formula (III).
Fig. 4 is a diagram showing the ¹H-NMR of the compound represented by formula (IV).
Fig. 5 is a diagram showing the ¹³C-NMR of the compound represented by formula (IV).
Fig. 6 is a diagram showing the mass spectrum of the compound represented by formula (IV).

### DETAILED DESCRIPTION OF THE INVENTION

A polymethoxyflavone used in the present invention is represented by the following general formula (I). 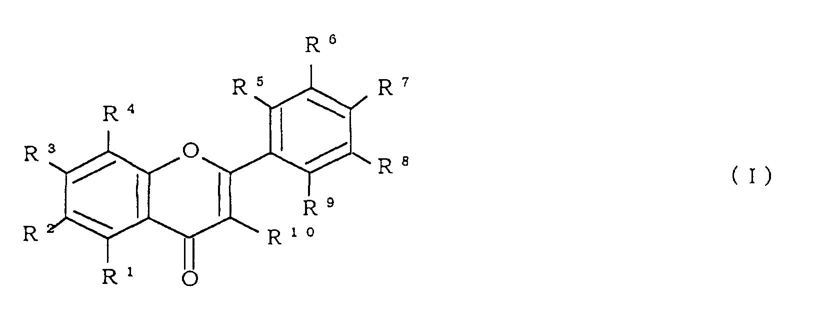 wherein each of R¹, R², R³, R⁴, R⁵, R⁶, R⁷, R⁸, R⁹, and R¹⁰ is selected from the group consisting of hydrogen atom, hydroxyl group, alkoxy group having 1 to 20 carbon atoms, alkyl group having 1 to 20 carbon atoms, alkenyl group having 2 to 20 carbon atoms, hydroxyalkyl group having 1 to 20 carbon atoms or a sugar residue, and at least four of R¹, R², R³, R⁴, R⁵, R⁶, R⁷, R⁸, R⁹, and R¹⁰ are methoxy groups. The structural feature is that the second and third positions of the flavonoid skeleton are reduced, and the compound has a total of four or more methoxy groups in the chromone ring and the benzene ring bonded to the second position. In other words, at least four of R¹ to R¹⁰ are methoxy groups. The positions of the methoxy groups may be either in the chromone ring or the benzene ring, but it is preferable that a larger number of methoxy groups are present in the chromone ring.

Examples of groups other than the methoxy groups include the following. As the alkoxy group having 1 to 20 carbon atoms, lower alkoxy groups having 1 to 6 carbon atoms are preferable, and methoxy groups, ethoxy groups, propoxy groups or the like are preferable.

As the alkyl group having 1 to 20 carbon atoms, lower alkyl groups having 1 to 6 carbon atoms are preferable, and methyl groups, ethyl groups, propyl groups or the like are preferable.

As the alkenyl group having 2 to 20 carbon atoms, lower alkenyl groups having 2 to 6 carbon atoms are preferable, and ethenyl groups, propenyl groups or the like are preferable.

As the hydroxyalkyl group having 1 to 20 carbon atoms, lower hydroxyalkyl groups having 1 to 6 carbon atoms are preferable, and hydroxymethyl groups, hydroxyethyl groups, hydroxypropyl groups or the like are preferable.

As the sugar residue, for example, monosaccharides such as glucose, galactose, fucose, xylose, mannose, rhamnose, fructose, arabinose, lyxose, ribose, allose, altrose, idose, talose, deoxyribose, quinovose and abequose; oligosaccharide residues where 2 to 4 of these monosaccharides are bonded, such as maltose, lactose, cellobiose, raffinose, xylobiose and sucrose can be used. Among these, residues of glucose, galactose, fucose, xylose, mannose, rhamnose, fructose are preferable.

Specific examples of the polymethoxyflavone include 3,5,6,7,8,3',4'-heptamethoxyflavone represented by formula (II), 5,6,7,8,3',4'-hexamethoxyflavone (commonly called nobiletin) represented by formula (III), 5,6,7,8,4'-pentamethoxyflavone (commonly called tangeretin) represented by formula (IV), and 5,7,8,3',4'-pentamethoxyflavone represented by formula (V).

Among these, 5,6,7,8,3',4'-hexamethoxyflavone (nobiletin) represented by formula (III), and 5,6,7,8,4'-pentamethoxyflavone (tangeretin) represented by formula (IV) are particularly preferable.

The polymethoxyflavone compound used in the present invention can be obtained by chemical synthesis, or can be obtained as a natural product from an extract with a solvent derived from a plant.

Examples of chemical synthesis include the method described in Indian Journal of Heterocyclic Chemistry Vol. 6. Jan-March 1997, pp.221-222. With this method, 5,6,7,8,3',4'- hexamethoxyflavone (formula (III):nobiletin ) can be synthesized.

Examples of a method by which polymethoxyflavone is obtained from an extract of a plant include extracting the polymethoxyflavone from the peel of plants of the Genus Citrus of the Family Rutaceae. Plants of the Genus Citrus of the Family Rutaceae, such as Citrus unshiu, Citrus poonensis, Citrus hassaku, Citrus lemon, Citrus tachibana, Citrus junos, Citrus sudachi, Citrus grandis, Citrus tangerina (tangerine), Citrus reticulate (mandarin orange), Citrus paradisi or the like contains polymethoxyflavone (Journal of Medicinal Plant Research Vol. 46, 162-166, (1982)). Among these, preferable plants are Citrus unshiu, Citrus tachibana, Citrus junos and Citrus sudachi, and more preferable plants are Citrus unshiu and Citrus tachibana.

Isolation and purification of polymethoxyflavone is performed by producing an extract from the peel of a plant of the Genus Citrus of the Family Rutaceae with an organic solvent, and separating the extract by column chromatography. This method will be described more specifically below.

First, peel of a plant of the Genus Citrus is subjected to extraction with a water-soluble alcohol such as methanol, ethanol, propanol, butanol, propylene glycol, 1,3-butylene glycol or the like; a solvent such as acetone, hexane, ethyl acetate, chloroform or the like; or a mixed solvent thereof. Among these, at least one solvent selected from the group consisting of methanol, ethanol, propanol, butanol, propylene glycol, 1,3-butylene glycol and ethyl acetate is preferable. More preferable is at least one solvent selected from the group consisting of methanol, ethanol, propylene glycol, 1,3-butylene glycol and ethyl acetate. When the extract liquid is concentrated, an extract (S1) comprising about 1 to 15 wt% of the desired polymethoxyflavone can be obtained. This extract (S1) can be further dried to a solid.

This extract (S1) is dissolved in ethyl acetate in an amount of twice or more times, preferably, 3 to 10 times the weight of the extract, water is added thereto, the mixture is stirred and separated into layers, and a water layer is removed. Then, the ethyl acetate is distilled off so as to obtain a dry solid product (S2).

Alternatively, the extract (S1) is dissolved in hexane and/or chloroform, stirred, and is left undisturbed. Thereafter, a precipitate is removed, and the supernatant was concentrated and a dry solid product (S3) can be obtained. As the solvent, hexane or chloroform can be used alone, however, a mixed solvent of hexane/chloroform is preferably used. The mixed solvent can be used in a volume proportion of 1/9 to 9/1, preferably, 3/7 to 7/3.

The obtained dry solid product (S2) or (S3) is preferably dissolved a suitable solvent and is subjected to liquid column chromatography so that polymethoxyflavone can be isolated and purified. In the liquid column chromatography, silica gel or alumina can be used as a filler, and a mixed solvent of hexane/ethanol in a volume proportion of 70/30 to 97/3 can be used as an eluent.

More specifically, isolation and purification of polymethoxyflavone from Citrus tachibana will be described. The dried peel of Citrus tachibana (herbal name: "kippi") is pulverized with a blender and dipped in a suitable solvent (e.g., 95% ethanol in an amount of five times the weight) for an appropriate period of time (e.g., five days) for extraction. The extract liquid is filtered and concentrated under reduced pressure so that a kippi extract (corresponding to S1 described above) can be obtained. A suitable amount of ethyl acetate is added to the obtained kippi extract to dissolve the kippi extract, followed by addition of water in an amount equal to that of the ethyl acetate and stirring, and the mixture was left undisturbed. After separating the mixture into layers, a water layer was removed. This operation (washing with water) was repeated plural times, preferably three times or more, and then ethyl acetate was distilled off to obtain a dry solid (corresponding to S2 described above). Alternatively, a suitable amount of mixed solvent of hexane/chloroform (in a volume proportion of 1/1) is added to the obtained kippi extract, and is stored undisturbed over night at an appropriate temperature (e.g., 4 °C). A precipitate is removed by separating means (e.g., centrifugation) and the supernatant is concentrated to obtain a dry solid (corresponding to S3 described above). This dry solid product (corresponding to S2 or S3 described above) is dissolved in an appropriate amount of a suitable solvent (e.g., hexane/ethanol (in a volume proportion of 85/15)), and subjected to fractional liquid chromatography using a silica gel column or an alumina column with a mixed solvent of hexane/ethanol as the eluent for fractionation. Thus, polymethoxyflavone can be isolated and purified.

The structure of the isolated and purified polymethoxyflavone can be determined by analysis means that is routinely used by those skilled in the art, such as the nuclear magnetic resonance (NMR) spectrum or the mass analysis spectrum.

In the present invention, the isolated polymethoxyflavone can be added alone or can be used in combination of two or more polymethoxyflavones, or can be added as a mixed product of chemical synthesis or plant extracts that contains a plurality of natural products.

The polymethoxyflavone is contained in an amount of 0.00005 to 10wt%, preferably 0.0001 to 7wt%, and more preferably 0.001 to 5wt% with respect to the total amount of the cosmetic compound. An amount of below 0.00005 wt% cannot provide a sufficient whitening effect, nor vitalize the skin or suppress wrinkles. An amount of more than 10wt% not only causes a problem in storage stability, but also is expensive.

Furthermore, the cosmetic composition of the present invention can comprise ascorbic acid and the derivatives thereof, kojic acid and the derivatives thereof, hydroquinone derivatives such as arbutin, placental extracts, ellagic acid and the derivatives thereof, which are known as whitening components. It is preferable that these whitening components are contained in an amount of 0.01 to 10wt%. The whitening effect can be synergistically enhanced by using these whitening components and polymethoxyflavone together.

The cosmetic composition of the present invention can contain a base, additives or the like that are usually used in cosmetics, as appropriate, depending on the type of the cosmetics in an amount that does not interfere with the performance of the cosmetic composition. For example, following substances can be contained in the cosmetic composition of the present invention: lower alcohols such as ethanol and isopropyl alcohol; polyhydric alcohols such as glycerol, 1,3-butylene glycol, propylene glycol, dipropylene glycol and polyethylene glycol; hydrocarbon oils such as liquid paraffin, liquid isoparaffin, squalane, veseline and solid paraffin; natural fats and oils such as beef tallow, lard and fish oil; synthetic triglyceride such as glycerol tri 2-ethylhexanoate; ester oils such as isopropyl myristate, isopropyl palmitate, cetyl palmitate, ethyl oleate, oleyl oleate and octyldodecyl myristate; waxes such as yellow bees wax and carnauba wax; silicone derivatives such as linear or cyclic dimethylpolysiloxane, polyether modified dimethylpolysiloxane, amino-modified dimethylpolysiloxane; oils such as ceramide, cholesterol, protein derivatives, lanoline, lanoline derivatives, lecithin; anionic surfactant such as soap, acylmethyl taurine salts, amide ether sulfuric ester salts; amphoteric surfactants such as amideamino acid salts, and amidopropyl dimethylbetaine; nonionic surfactants such as polyoxyethylene alkyl ethers, polyethylene glycol fatty acid esters, sorbitan fatty acid esters, polyoxyethylene sorbitan fatty acid esters, polyoxyethylene hydrogenated castor oil, polyglycerol fatty acid esters, polyoxyethylene glycerol fatty acid esters, glycerol mono-fatty acid esters, alkyl polyglucoside and alkanolamide; cationic surfactants such as alkyltrimethylammonium chloride; semipolar surfactants such as alkyl dimethylamine oxides; water-soluble polymers such as algic acid, carboxyvinyl polymer, carboxymethylcellulose, hydroxypropylmethyl-cellulose, hydroxyethyl cellulose, xanthan gum and hyaluronic acid; organic or inorganic salts such as pyrrolidone carboxylic acid salts, citric acid salts, malic acid salts and sodium chloride; acids or alkalis that are pH regulators; antiinflammatory agents; germicidal agents; chelating agents; antioxidants; ultraviolet absorbers; natural extracts derived from animals and plants; and pigments and fragrances.

The cosmetic composition of the present invention can be preferably used in the form of lotions, milky lotions, oil-in-water creams, water-in-oil creams or the like.

### Examples

Next, the present invention will be described by way of examples, but the present invention is not limited by these examples.

### (Production Example 1: Isolation of polymethoxyflavone)

First, 10kg of dried peel of Citrus tachibana (herbal name: "kippi") was pulverized with a blender, and immersed for extraction for 5 days in 95% ethanol (first grade) in an amount of 5 times the weight of the dried peel. An extract liquid was filtered and concentrated under reduced pressure, and thus 550g of a kippi extract were obtained. The kippi extract was dissolved in ethyl acetate in an amount of 5 times the weight of the extract, followed by addition of water in an amount equal to that of the ethyl acetate and stirring, and the mixture was left undisturbed. After separating the mixture into layers, a water layer was removed (washing with water). This operation (washing with water) was repeated three times, and then ethyl acetate was distilled off. Thus, 300g of dry solid product were obtained. Furthermore, this dry solid product was dissolved in a mixed solution of hexane / ethanol (in a volume proportion of 85/15) in an amount of twice the weight of the dry solid product, and thus a dissolved solution was obtained. All the dissolved solution was charged to a column (φ 30cm, with a length of 1m) filled with silica gel. Hexane in a volume of twice the volume of the column was allowed to flow in the column, and then, using a mixed solvent of hexane/ethanol (in a volume proportion of 90/10) as an eluent, the elution was fractionated into 1L fractions. When each fraction was analyzed by silica gel thin layer chromatography using a hexane/ethanol mixed solution (volume proportion of 90/10) as a developer, the presence of compounds 1 to 4 was confirmed. The fractions containing these compounds were mixed, and the solvent was distilled off to obtain the compounds 1 to 4. The yields of the compounds were as follows: 7g for compound 1, 63g for compound 2, 33g for compound 3, and 13g for compound 4.

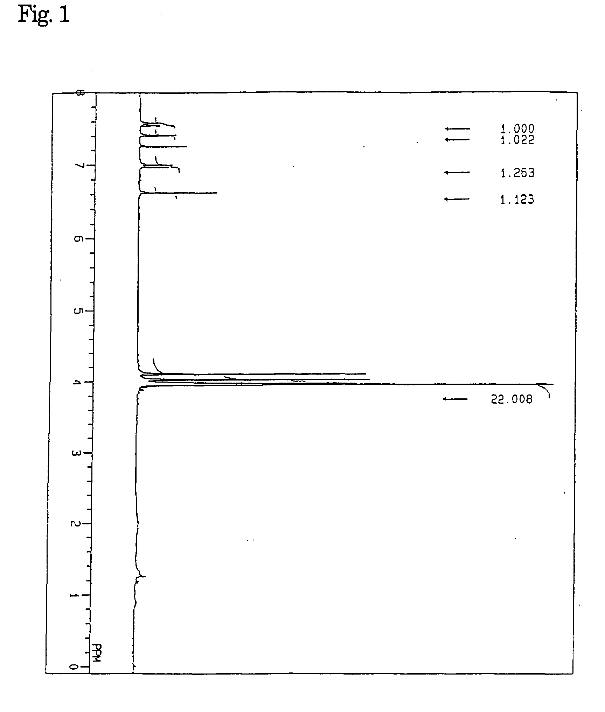

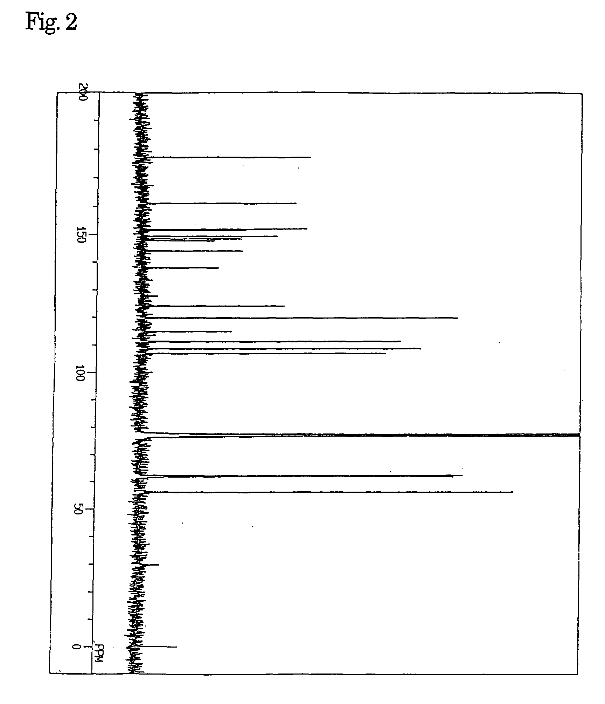

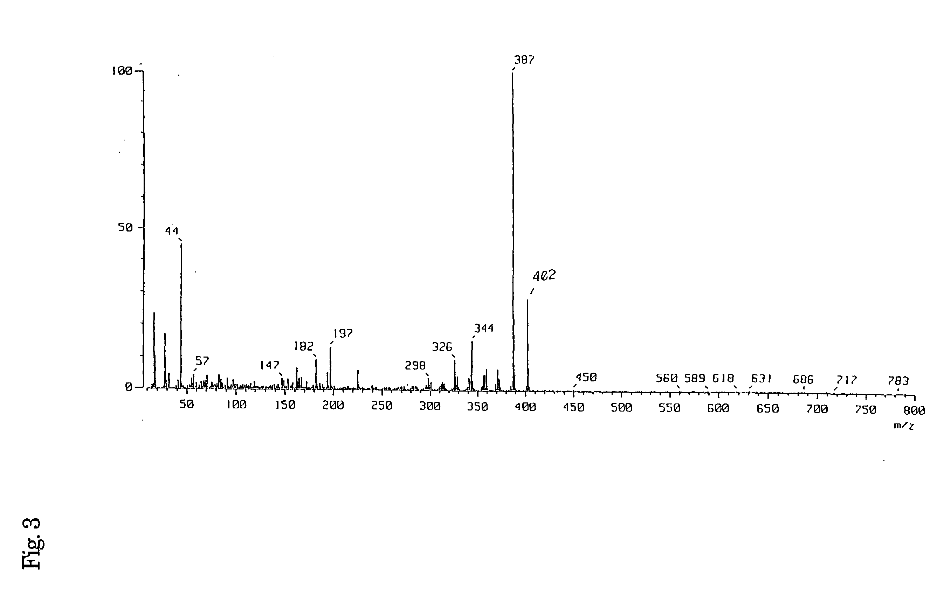

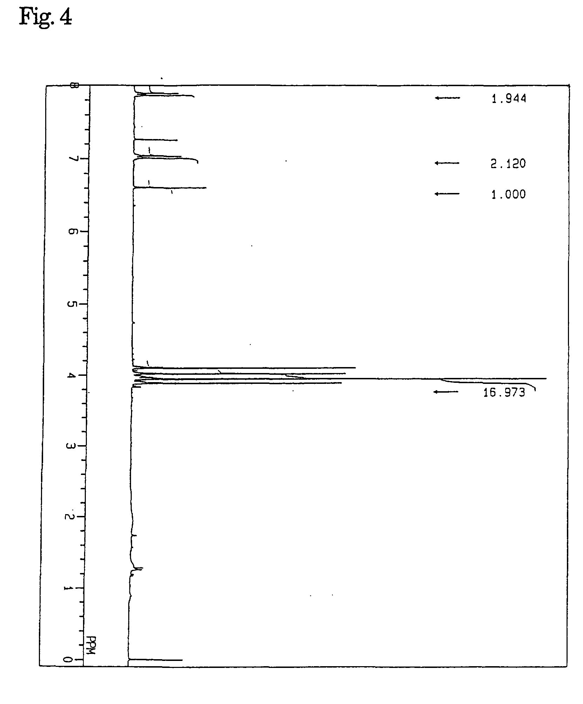

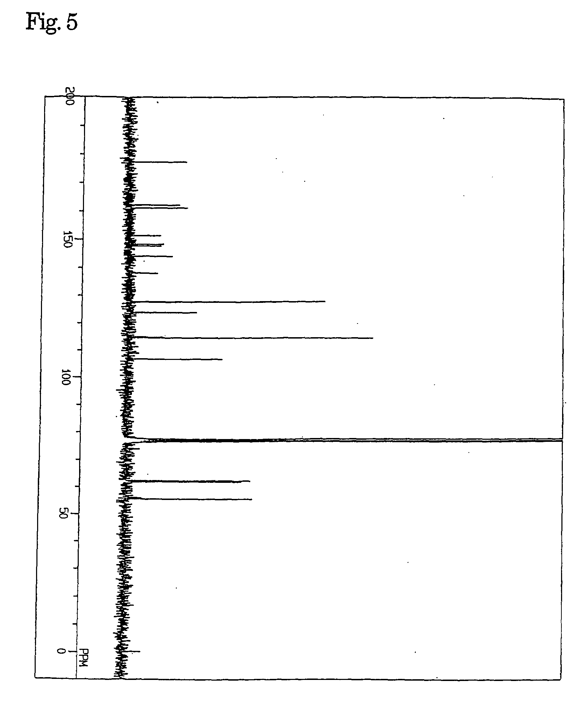

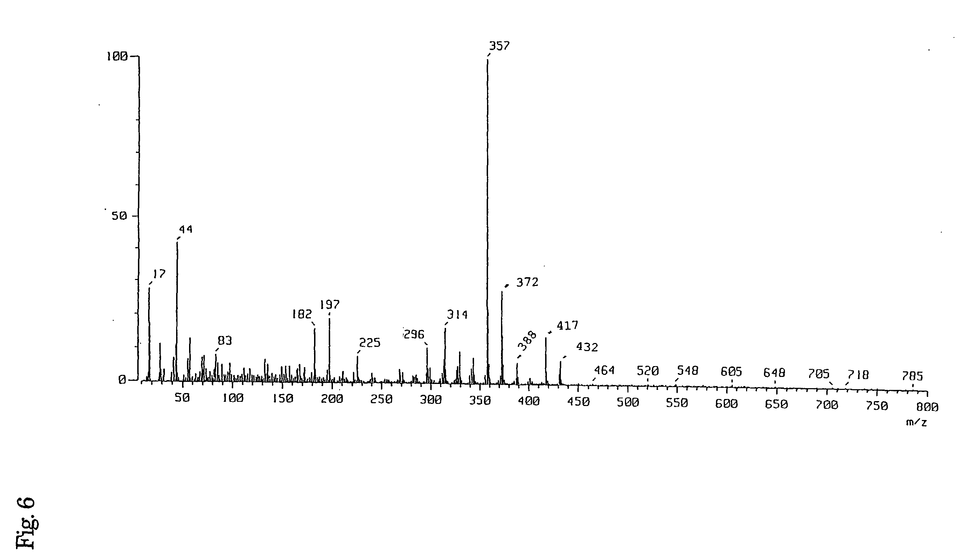

Each of the obtained compounds 1 to 4 was analyzed by the nuclear magnetic resonance (NMR) spectrum and the mass analysis (MS) spectrum. Figs. 1 to 3 show the ¹H-NMR, ¹³C-NMR and MS spectra of the compound 2, and Figs 4 to 6 show the ¹H-NMR, ¹³C-NMR and MS spectra of the compound 3.

When these data are compared with the data described in references (Natural Medicines 51(3), 190-193(1997), Chem. Pharm. Bull. 37, 1092(1989), Pharmacological Magazine (YAKUGAKU ZASSHI) 116(3), 244-250 (1996) and Tetrahedron, 16(8), 64(1964), the compound 2 was identified as 5,6,7,8,3',4'-hexamethoxyflavone (formula (III): nobiletin). The compound 3 was identified as 5,6,7,8,4'-pentamethoxyflavone (formula (IV): tangeretin).

The structures of the other compounds (compound 1 and compound 4) were determined by comparing their ¹H-NMR, ¹³C-NMR and MS spectra with the ¹H-NMR, ¹³C-NMR and MS spectra of the compounds represented by formulae (III) and (IV). As a result, the compound 1 was identified as 3,5,6,7,8,3',4'-heptamethoxyflavone (formula (II)), and the compound 4 was identified as 5,7,8,3',4'-pentamethoxyflavone (formula (V)).

### (Production Example 2: Isolation of polymethoxyflavone)

First, 10kg of dried peel of Citrus tachibana (herbal name: "kippi") was pulverized with a blender, and immersed for extraction for 5 days in 95% ethanol (first grade) in an amount of 5 times the weight of the dried peel. An extract liquid was filtered and concentrated under reduced pressure, and thus 560g of a kippi extract were obtained. A mixed solvent of hexane/ chloroform (in a volume proportion of 1/1) in an amount of five times the weight was added to the kippi extract, and the mixture was stirred for about 30 min. Thereafter, the mixture was left undisturbed at 4 °C over night, and a supernatant was collected by decantation, followed by distillation of the solvent. Thus, 320g of dry solid product were obtained. This dry solid product was separated and purified by being subjected to column chromatography in the same manner as in Production Example 1, and thus compounds 1 to 4 were collected. The yields of the compounds were as follows: 6g for compound 1, 65g for compound 2, 35g for compound 3, and 14g for compound 4. The results of analysis revealed that the compounds 1 to 4 correspond to the compounds (II) to (V), respectively.

### (Confirmation of whitening effect-1: suppression of melanin formation)

Using the obtained compounds of formulae (II) to (V), the effect of suppressing melanin production of human melanoma cell (HM3KO) was examined in vitro. More specifically, HM3KO was cultured to about 5 × 10⁵ by a conventional method, collected by centrifugation to obtain pellets, and inoculated the pellet to a culture dish of 10cm diameter containing an Eagle's medium supplemented with 10% fetal bovine serum and cultured at 37 °C for 24 hours. Thereafter, each polymethoxyflavone of formulae (II) to (V) was added, or kojic acid or arbutin for comparison was added thereto so that the final addition concentration was 10µM, followed by cultivation for 6 days. After the cultivation, cells were collected by centrifugation, and 1ml of 2N sodium hydroxide aqueous solution was added thereto to obtain cell lysate. The absorbance in a wavelength of 410nm of the cell lysate was measured with a spectrophotometer. Here, the absorbance of the cell lysate to which a sample is not added is defined as 100% of the melanin production ratio, and the melanin production ratios are shown as relative values, measuring the absorbance of each cell lysate. Table 1 shows the results.

**Table 1**

| Sample | Sample concentration in medium (*µ*M) | Melanin production ratio (% of control) |
|---|---|---|
| (no addition) | 0 | 100 |
| 3,5,6,7,8,3',4'-heptamethoxyflavone : formula (II) | 10 | 65 |
| 5,6,7,8,3',4'-hexamethoxyflavone : formula (III) | 10 | 35 |
| 5,6,7,8,4'-pentamethoxyflavone : formula (IV) | 10 | 55 |
| 5,7,8,3',4'-pentamethoxyflavone : formula (V) | 10 | 60 |
| Kojic acid | 10 | 96 |
| Arbutin | 10 | 97 |

The results of Table 1 indicate that all of the polymethoxyflavones of formulae (II) to (V) significantly suppressed melanization of HM3KO. This effects are far beyond those of kojic acid and arbutin.

### (Confirmation of whitening effect-2: suppression of UV-induced pigmentation)

Using brown guinea pigs, the suppression of UV-induced pigmentation was examined in vivo. More specifically, the backs of the brown guinea pigs were shaved and covered with an ultraviolet shielding plate provided with 6 rectangular holes of 2 × 2cm, followed by irradiation of UV (0.5J/cm²) to induce pigmentation. Thereafter, 40 µL of 70% (W/W) ethanol aqueous solution containing each of polymethoxyflavone (nobiletin) of formula (III) or polymethoxyflavone (tangeretin) of formula (IV) in a concentration shown in Table 2 were applied twice a day. Then, the degrees of pigmentation before and after the application were measured. Table 2 shows the results.

**Table 2**

| Sample | Sample concentration (%) | Pigmentation degree ΔL | | |
|---|---|---|---|---|
| | | Before irradiation | After 20 days | After 40 days |
| (no addition) | 0 | 0 | -7.8 | -5.2 |
| 5,6,7,8,3',4'- hexamethoxyflavone: formula (III) | 4 | 0 | -5.1 | -1.9 |
| 5,6,7,8,3',4'- hexamethoxyflavone: formula (III) | 0.4 | 0 | -5.5 | -3.2 |
| 5,6,7,8,4'-pentamethoxyflavone: formula (IV) | 4 | 0 | -5.6 | -3.3 |
| 5,6,7,8,4'-pentamethoxyflavone: formula (IV) | 0.4 | 0 | -6.1 | -3.5 |

In Table 2, the pigmentation degree Δ L was obtained by measuring a L value before UV irradiation, which is defined as 0, with a spectrophotometric colorimeter, and obtaining the difference between the L values before and after UV irradiation. A lower value ΔL corresponds to a higher pigmentation degree.

Table 2 indicates that the polymethoxyflavone used in the present invention significantly suppress the induction of pigmentation of brown guinea pigs.

### Examples 1 to 4 and Comparative Examples 1 to 3

Cosmetic compositions for skin lotion containing components shown in Table 4 were prepared by using five components shown in Table 3 as common components. The polymethoxyflavones used were the polymethoxyflavones of formulae (III) and (IV) that were isolated and purified in the production examples.

**Table 3**

| Commonly added components | | |
|---|---|---|
| | Components | Added amount (wt%) |
| 1 | Trisodium citrate dihydrate | 0.3 |
| 2 | Ethanol | 3 |
| 3 | Methylparaben | 0.1 |
| 4 | Phenoxyethanol | 0.2 |
| 5 | Sodium sulfate anhydride | 0.1 |
| Total | | 3.7 |

The obtained cosmetic compositions were evaluated by the following method. Table 4 shows the results.

### (1) Whitening effect

Evaluation was carried out with 50 women (in their 20's and 30's) as test persons, and the cosmetic compositions were used twice a day, that is, in the morning and at night, for 2 months. Then, the improvement degree in "liver spots" and "freckles" after use was determined by eyesight with the following criteria.

10 points: when it was determined that the composition was evidently effective.

5 points: when it was determined that the composition was slightly effective.

0 point: when it was determined that the composition was not effective at all.

The average of the points of the 50 women was obtained, and a cosmetic composition for which the average was 5 points or more was defined as a cosmetic composition having high whitening effect.
G: high whitening effect (5 points or more in average)
P: low whitening effect (below 5 points in average)

### (2) Moisture of the skin

Evaluation was carried out with 20 women (of the age between 22 and 32) as test persons, and the cosmetic compositions were used after washing their faces. Then, moisture of the skin immediately after use and 2 hours later were determined with the following criteria.

2 points: when it was perceived that the moisture of the skin was sufficient immediately after use, and the skin was still moist 2 hours later.

1 point: when it was perceived that the moisture of the skin was slightly not sufficient immediately after use, or the skin was slightly dry 2 hours later.

0 point: when it was perceived that the moisture of the skin was not sufficient.

The average of the points of the 20 women was obtained, and a cosmetic composition for which the average was 1.5 points or more was defined as a cosmetic composition having a high effect of allowing the skin to retain moisture.
G: high effect of allowing the skin to retain moisture (1.5 points or more in average)
P: low effect of allowing the skin to retain moisture (below 1.5 points in average)

### (3) Vitality of the skin

Evaluation was carried out with 20 women (of the age between 22 and 32) as test persons, and the cosmetic compositions were used after washing their faces. Then, the vitality of the skin was determined with the following criteria.

2 points: when it was perceived that the skin was vitalized.

1 point: when it was perceived that the skin was slightly vitalized.

0 point: when it was perceived that the skin was not vitalized.

The average of the points of the 20 women was obtained, and a cosmetic composition for which the average was 1.5 points or more was defined as a cosmetic composition having a high effect of vitalizing the skin.
G: high effect of vitalizing the skin (1.5 points or more in average)
P: low effect of vitalizing the skin (below 1.5 points in average)

### (4) Wrinkle suppressing effect

Evaluation was carried out with 20 women (of the age between 24 and 35) as test persons, and the cosmetic compositions were used twice a day (in the morning and at night) for two weeks in a row. Then, the wrinkle suppressing effect was determined with the following criteria.

2 points: when it was perceived apparently that wrinkles were not conspicuous any more.

1 point: when it was perceived that wrinkles were slightly not conspicuous.

0 point: when it was perceived that there was no wrinkle suppressing effect.

The average of the points of the 20 women was obtained, and a cosmetic composition for which the average was 1.5 points or more was defined as a cosmetic composition having a high effect of suppressing wrinkles.
G: high effect of suppressing wrinkles (1.5 points or more in average)
P: low effect of suppressing wrinkles (below 1.5 points in average)

### (5) Storage stability

The cosmetic compositions were sealed in transparent glass containers, and stored at 0°C, 25°C, and 40°C for three months. The appearance thereof was observed, and evaluation was carried out with the following criteria.
G: Good stability (there is no change in the appearance at any temperatures)
S: Slightly poor stability (A sediment or precipitate slightly occurs, or slight coloring occurs at some temperatures)
P: Poor stability (A sediment or precipitate occurs or separation occurs at some temperatures, or coloring is significant.)

Table 4 shows the results.

**Table 4**

| | Examples (wt%) | | | | Comparative Examples (wt%) | | |
|---|---|---|---|---|---|---|---|
| | 1 | 2 | 3 | 4 | 1 | 2 | 3 |
| a 5,6,7,8,3',4'-hexamethoxyflavone: formula (III) | 2 | --- | 0.5 | 0.3 | --- | --- | --- |
| a. 5,6,7,8,4'-pentamethoxyflavone: formula (IV) | --- | 2 | --- | 0.1 | --- | --- | --- |
| Placental extract | --- | --- | --- | --- | --- | 0.5 | --- |
| Ascorbic acid magnesium phosphate salt | --- | --- | 0.5 | --- | --- | 0.5 | 3 |
| Glycerol | --- | 2 | 2 | 2 | 2 | 2 | 2 |
| Dipropylene glycol | --- | 3 | 3 | 3 | 3 | 3 | 3 |
| Citrate monohydrate | 0.1 | 0.1 | --- | 0.1 | 0.1 | --- | -- |
| Commonly added components | 3.7 | | | | | | |
| Purified water | the rest | | | | | | |
| Total | 100 | | | | | | |
| (1) Whitening effect | G (7.0) | G (6.3) | G (7.5) | G (7.2) | P (1.8) | P (2.2) | P (3.7) |
| (2) Skin moisture | G (1.7) | G (1.8) | G (1.9) | G (1.8) | P (1.2) | P (1.4) | P (1.4) |
| (3) Skin vitality | G (1.7) | G (1.9) | G (1.8) | G (1.7) | P (1.3) | P (1.3) | P (1.3) |
| (4) Wrinkle suppressing effect | G (1.8) | G (1.9) | G (1.8) | G (1.7) | P (1.2) | P (1.2) | P (1.3) |
| (5) Storage stability | G | G | G | G | G | G | P |

According to Examples 1 to 4, the lotions containing polymethoxyflavones of the present invention are excellent in the whitening effect, allow the skin to retain moisture for a long time, and vitalize the skin, and suppress wrinkles, and have excellent storage stability. On the other hand, Comparative Examples 1 to 3 cannot provide cosmetic compositions with adequate performance. More specifically, in Comparative Example 1, since no polymethoxyflavone is contained, almost no whitening effect is provided. In Comparative Example 2 where placental extract is used instead of the polymethoxyflavone, the whitening effect is weak, and there is no effect on the skin moisturization, the skin vitalization, and the suppression of wrinkles. In Comparative Example 3 where ascorbic acid salt is used instead of the polymethoxyflavone, the whitening effect is weak, and there is no effect on the skin moisturization, the skin vitalization, and the suppression of wrinkles, and the storage stability is also problematic.

### Examples 5 to 7

Cosmetic whitening compositions for oil-in-water milky lotion shown in Table 8 were prepared by using the 12 components shown in Table 5 as common components.

**Table 5**

| Commonly added components | | |
|---|---|---|
| | Components | Added amount (wt%) |
| 1 | Xanthan gum (manufactured by Dainippon Pharmaceutical Co., Ltd., "ECHO GUM T") | 0.1 |
| 2 | Carboxyvinyl polymer (BFGoodrich Co., Ltd., "carbopol 940") | 0.12 |
| 3 | Ethanol | 5 |
| 4 | Purified sunflower oil | 5 |
| 5 | Squalane | 3 |
| 6 | Octyldodecyl myristate | 2 |
| 7 | Polyoxyethylene(32mol)monostearate | 0.5 |
| 8 | Polyoxyethylene (20mol) stearyl ether | 0.7 |
| 9 | Glycerol monostearate | 1 |
| 10 | Methylparaben | 0.1 |
| 11 | Butylparaben | 0.05 |
| 12 | Phenoxyethanol | 0.2 |
| Total | | 17.77 |

The obtained milky lotions were evaluated in the same manner as in Examples 1 to 4. Table 8 shows the results.

### Examples 8 and 9

Cosmetic skin care compositions for oil-in-water creams shown in Table 8 were prepared by using the 12 components shown in Table 6 as common components.

**Table 6**

| Commonly added components | | |
|---|---|---|
| | Components | Added amount (wt%) |
| 1 | Purified olive oil | 8 |
| 2 | Squalane | 3 |
| 3 | Octyldodecyl myristate | 2 |
| 4 | Dimethylpolysiloxane (100CS) | 1 |
| 5 | Purified yellow wax | 3 |
| 6 | Polyoxyethylene (20mol) sorbitan monostearate | 1 |
| 7 | Polyoxyethylene(75mol)monostearate | 1 |
| 8 | Glycerol monostearate | 2 |
| 9 | Tocopherol acetate | 0.05 |
| 10 | Methylparaben | 0.2 |
| 11 | Propylparaben | 0.1 |
| 12 | Butylparaben | 0.1 |
| Total | | 21.45 |

The obtained oil in water creams were evaluated in the same manner as in Examples 1 to 4. The storage stability was evaluated with the method shown in (5') below.

### (5') Storage stability

The cosmetic compositions were sealed in transparent glass containers, and stored at - 5°C, 25°C, and 45°C for one month. Then, their state was examined, and evaluation was carried out with the following three criteria.
G: Good stability (there is no change in the appearance at any temperatures, and there is no aggregation or the like)
S: Slightly poor stability (slight precipitation occurs, or slight separation occurs at some temperatures, or aggregations or lumps are formed slightly.)
P: Poor stability (Apparently, a precipitate occurs or separation occurs at some temperatures, or aggregations or lumps appear.)

Table 8 shows the results.

### Examples 10 and 11

Cosmetic skin care compositions for water-in-oil creams shown in Table 8 were prepared by using the 12 components shown in Table 7 as common components.

**Table 7**

| Commonly added components | | |
|---|---|---|
| | Components | Added amount (wt%) |
| 1 | Purified jojoba oil | 3 |
| 2 | Purified sunflower oil | 6 |
| 3 | Dimethylpolysiloxane (100CS) | 1 |
| 4 | Squalane | 4 |
| 5 | Octyldodecyl myristate | 5 |
| 6 | Glycerol monooleate | 1.5 |
| 7 | Diglycerol monooleate | 0.2 |
| 8 | Tocopherol acetate | 0.1 |
| 9 | Methylparaben | 0.2 |
| 10 | Propylparaben | 0.1 |
| 11 | Butylparaben | 0.1 |
| 12 | Magnesium sulfate (heptahydrate) | 0.5 |
| Total | | 21.7 |

The obtained water-in-oil creams were evaluated in the same manner as in Examples 8 and 9.

Table 8 shows the results.

**Table 8**

| | Examples (wt%) | | | | | | |
|---|---|---|---|---|---|---|---|
| | o/w milky lotion | | | o/w cream | | w/o cream | |
| | 5 | 6 | 7 | 8 | 9 | 10 | 11 |
| a5,6,7,8,3',4'-hexamethoxyflavone: formula(III) | 1 | - | 0.5 | 2 | 0.5 | 1 | 2 |
| a.5,6,7,8,4'-pentamethoxyflavone: formula(IV) | - | 2 | - | - | 1 | - | 1 |
| Glycerol | 2 | 2 | 2 | 2 | 2 | 2 | 2 |
| Dipropylene glycol | 2 | 2 | 2 | 3 | 3 | 3 | 3 |
| Polyethylene glycol 1540 | 2 | 2 | 2 | - | - | - | - |
| Ascorbic acid magnesium phosphate salt | - | - | - | 0.5 | - | - | - |
| L-arginine | 0.1 | 0.1 | 0.1 | - | - | - | - |
| Cetanol | 2 | - | - | 3 | - | - | 3 |
| Behenyl alcohol | - | 1 | 1 | - | 3 | 2 | - |
| Decamethylcyclosiloxane | - | - | - | 3 | - | - | - |
| Commonly added components | 17.77(table 5) | | | 21.45(table 6) | | 21.7(table 7) | |
| Purified water | the rest | | | | | | |
| Total | 100 | | | | | | |
| (1) Whitening effect | G (7.1) | G (6.4) | G (7.8) | G (7.2) | G (6.8) | G (7.6) | G (8.1) |
| (2) Skin moisture | G (1.9) | G (1.9) | G (1.8) | G (1.9) | G (1.9) | G (2.0) | G (2.0) |
| (3) Skin vitality | G (1.8) | G (1.9) | G (1.9) | G (1.8) | G (1.9) | G (1.8) | G (1.8) |
| (4) Wrinkle suppressing effect | G (1.9) | G (2.0) | G (1.9) | G (1.9) | G (1.9) | G (1.9) | G (1.8) |
| (5) Storage stability | G | G | G | - | - | - | - |
| (5')Storage stability | - | - | - | G | G | G | G |

According to Examples 5 to 7, all the cosmetic compositions (milky lotions) of the present invention have an excellent whitening effect, allow the skin to retain moisture for a long time, vitalize the skin, suppress wrinkles, and have excellent storage stability.

According to Examples 8 and 9, all the cosmetic skin care compositions (creams) of the present invention have an excellent whitening effect, allow the skin to retain moisture for a long time, vitalize the skin, suppress wrinkles, and have excellent storage stability.

According to Examples 10 and 11, all the cosmetic compositions (creams) of the present invention have an excellent whitening effect, allow the skin to retain moisture for a long time, vitalize the skin, suppress wrinkles, and have excellent storage stability.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A cosmetic composition comprising 0.00005 to 10wt% of polymethoxyflavone represented by formula (I): 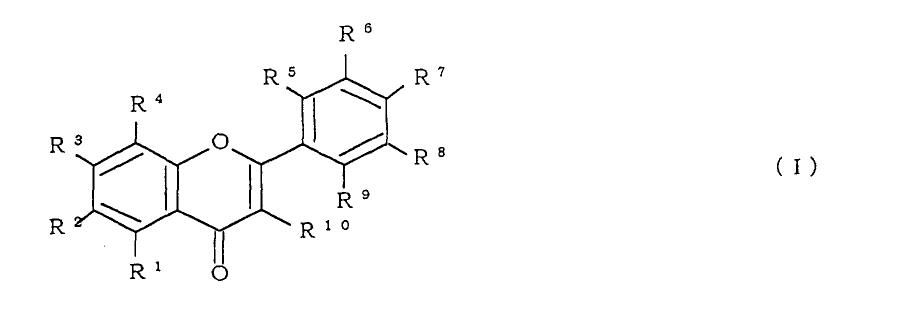 wherein each of R¹, R², R³, R⁴, R⁵, R⁶, R⁷, R⁸, R⁹, and R¹⁰ is selected from the group consisting of hydrogen atom, hydroxyl group, alkoxy group having 1 to 20 carbon atoms, alkyl group having 1 to 20 carbon atoms, alkenyl group having 2 to 20 carbon atoms, hydroxyalkyl group having 1 to 20 carbon atoms or a sugar residue, and at least four of R¹, R², R³, R⁴, R⁵, R⁶, R⁷, R⁸, R⁹, and R¹⁰ are methoxy groups.

2. The cosmetic composition of claim 1, wherein the polymethoxyflavone comprises at least one compound selected from the group consisting of compounds represented by formulae (II) to (V):

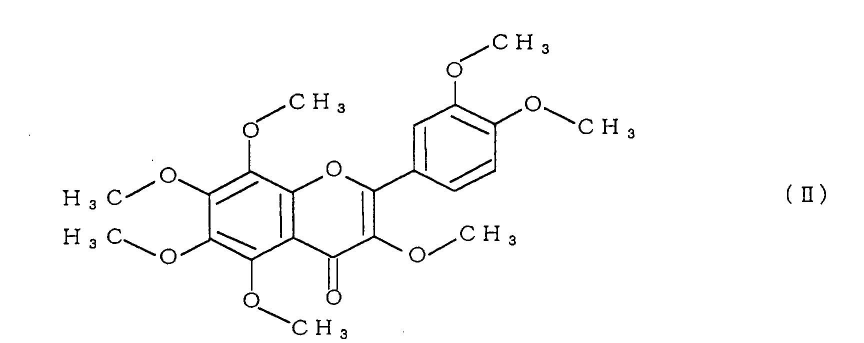

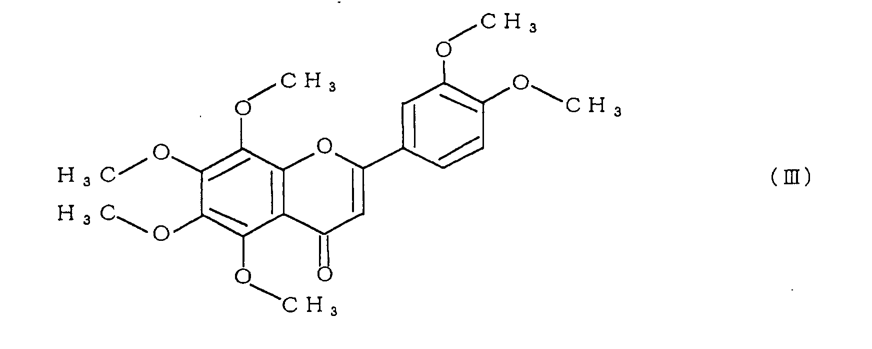

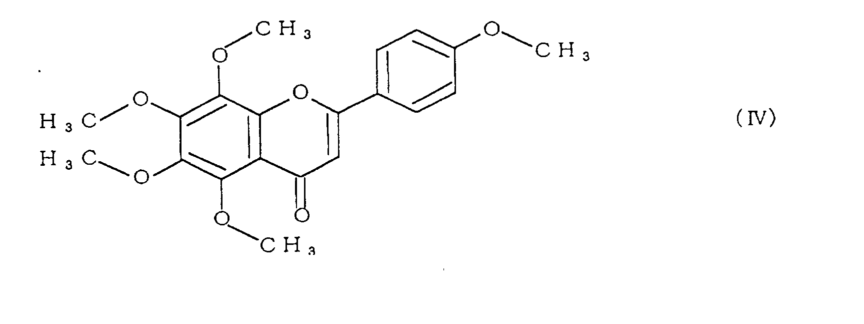

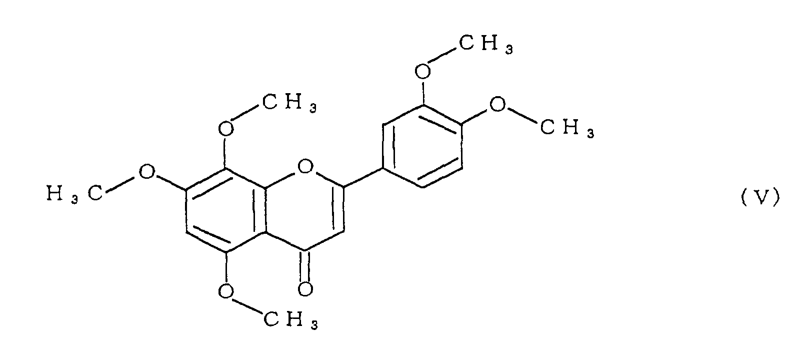

3. The cosmetic composition of claim 2, wherein the polymethoxyflavone comprises at least one compound selected from the group consisting of 5,6,7,8,3',4'-hexamethoxyflavone and 5,6,7,8,4'-pentamethoxyflavone.

4. A method for isolating and purifying polymethoxyflavone comprising the steps of:
subjecting peel of a plant of the Genus Citrus of the Family Rutaceae to extraction with at least one solvent selected from the group consisting of methanol, ethanol, propanol, butanol, ethyl acetate, acetone, propylene glycol, and 1, 3-butylene glycol to obtain an extract (S1);
dissolving the extract (S1) in ethyl acetate, adding water thereto, stirring, separating into layers, removing a water layer, distilling off the ethyl acetate to obtain a dry solid product (S2); and
dissolving the dry solid product (S2) in a solvent, and subjecting it to liquid column chromatography.

5. A method for isolating and purifying polymethoxyflavone comprising the steps of:
subjecting peel of a plant of the Genus Citrus of the Family Rutaceae to extraction with at least one solvent selected from the group consisting of methanol, ethanol, propanol, butanol, ethyl acetate, acetone, propylene glycol, and 1, 3-butylene glycol to obtain an extract (S1);
dissolving the extract (S1) in hexane and/or chloroform, removing a precipitate, distilling off the hexane and/or chloroform to obtain a dry solid product (S3); and
dissolving the dry solid product (S3) in a solvent, and subjecting it to liquid column chromatography.

6. The method of claim 4 or 5, wherein the liquid column chromatography uses silica gel and/or alumina as a filler, and a mixed solution of hexane/ethanol in a volume proportion of 70/30 to 97/3 as an eluent.
